(19) Europäisches Patentamt
European Patent Office
Office européen des brevets

(11) EP 1 887 085 A1

(12) EUROPEAN PATENT APPLICATION
published in accordance with Art. 153(4) EPC

(43) Date of publication:
13.02.2008 Bulletin 2008/07

(21) Application number: 06756711.5

(22) Date of filing: 30.05.2006

(51) Int Cl.:
*C12P 7/24* (2006.01) *C12N 15/09* (2006.01)

(86) International application number:
PCT/JP2006/310715

(87) International publication number:
WO 2006/129628 (07.12.2006 Gazette 2006/49)

(84) Designated Contracting States:
AT BE BG CH CY CZ DE DK EE ES FI FR GB GR HU IE IS IT LI LT LU LV MC NL PL PT RO SE SI SK TR

(30) Priority: 31.05.2005 JP 2005158393

(71) Applicant: Kaneka Corporation
Kita-ku
Osaka-shi, Osaka 530-8288 (JP)

(72) Inventors:
- YOSHIDA, Takashi,
KANEKA CORPORATION
Takasago-shi, Hyogo,
676-8688 (JP)
- TAOKA, Naoaki,
KANEKA CORPORATION
Takasago-shi, Hyogo 676-8688 (JP)

(74) Representative: Vossius & Partner
Siebertstrasse 4
81675 München (DE)

# (54) PROCESS FOR PRODUCTION OF OPTICALLY ACTIVE 2-SUBSTITUTED PROPANAL DERIVATIVE

(57) The present invention relates to a process for producing an optically active 2-substituted propanal derivative, and more particularly, a process for producing an optically active 2-substituted propanal derivative which comprises stereoselectively reducing a carbon-carbon double bond of a 2-substituted acrolein derivative by using an enzyme source capable of stereoselectively reducing said carbon-carbon double bond.

According to the present invention, it becomes possible to produce an optically active 2-substituted propanal derivative, in particular an optically active 2-alkylpropanal derivative, which is useful as an intermediate of pharmaceutical products, sweetening agents, etc., in a convenient manner from inexpensive and easily available materials.

EP 1 887 085 A1

**Description**

TECHNICAL FIELD

The present invention relates to a process for producing an optically active 2-substituted propanal derivative, in particular an optically active 2-alkylpropanal derivative, which is useful as an intermediate of pharmaceutical products, sweetening agents, etc. More particularly, the present invention relates to a process for producing an optically active 2-substituted propanal derivative, in particular an optically active 2-alkylpropanal derivative, which comprises stereoselectively reducing a 2-substituted acrolein derivative, which is available at low cost, by using an enzyme source capable of stereoselectively reducing a carbon-carbon double bond of the derivative.

(Reference of related applications)

The whole disclosure including the description, the claims, the drawings and the abstract in Japanese Patent 2005-158393 (filed on 2005, May 31) is herein incorporated in the present application by reference.

BACKGROUNG ART

At present, methods mentioned below are known as processes for producing an optically active 2-substituted propanal derivative.

1) A method for synthesizing (R)-2-methylpentanal with the optical purity of 94%ee by asymmetric alkylation of propanal using a proline derivative as an asymmetric auxiliary group (Non-Patent Document 1).
2) A method for synthesizing optically active 2-methylpentanal which comprises reducing 2-methyl-2-pentenal by a microorganism using *Beauveria Sulfurescens* to obtain optically active 2-methyl-1-pentanol and oxidizing the same. (Non-Patent Document 2).

Non-Patent Document 1: New Journal of Chemistry, 24(12), 973-975 (2000)
Non-Patent Document 2: Tetrahedron, 37(22), 3825-3829 (1981)

SUMMARY OF THE INVENTION

The above method 1) requires use of an expensive asymmetric auxiliary group for the asymmetric alkylation reaction. And by the above method 2), a large amount of 2-methyl-2-pentene-1-ol is produced as a byproduct.
In view of the above-mentioned state of the art, the present invention has for its object to provide a process for producing an optically active 2-substituted propanal derivative, in particular an optically active 2-alkylpropanal derivative, which is useful as an intermediate of pharmaceutical products, sweetening agents, etc., in a convenient manner from inexpensive and easily available materials.
The present inventors had made intensive investigations for solving the above-mentioned subjects, and as a result, found a process for producing, in a convenient manner, an optically active 2-substituted propanal derivative which comprises stereoselectively reducing a 2-substituted acrolein derivative, which is available at low cost, by using an enzyme source capable of stereoselectively reducing a carbon-carbon double bond of the derivative.
That is, one of the features of the present invention is to provide
a process for producing an optically active 2-substituted propanal derivative represented by the general formula (2):

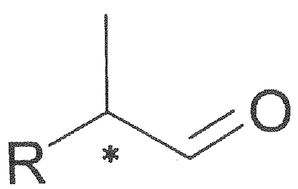
(2)

(in the formula, R is a methyl group having a substituent, an alkyl group containing 2 to 10 carbon atoms which may optionally be substituted, or an aralkyl group containing 5 to 15 carbon atoms which may optionally be substituted, and * is an asymmetric carbon)
which comprises reacting a 2-substituted acrolein derivative represented by the general formula (1) :

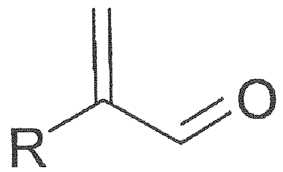

(1)

(in the formula, R is as mentioned above) with an enzyme source capable of stereoselectively reducing the carbon-carbon double bond of said 2-substituted acrolein derivative (1).

(Effect of the invention)

Other features of the present invention and those effects are shown in the following embodiment and figure.

By the above-mentioned process of the present invention, an optically active 2-substituted propanal derivative which is useful as an intermediate of pharmaceutical products, sweetening agents, etc. can be produced in a convenient manner from inexpensive materials.

BRIEF DESCRITPION OF THE DRAWING

Fig. 1 is a schematic view of the process for producing a recombinant vector pTSYE2G1 and the constitution thereof.

DETAILED DESCRIPTION OF THE INVENTION

1. 2-substituted acrolein derivative (starting material)

First, compounds involved with the embodiment of the present invention are described. A starting material used in the process according to the embodiment of the invention is the 2-substituted acrolein compound represented by the above formula (1). As a substituent R in the formula (1), there may be mentioned a methyl group having a substituent, an alkyl group containing 2 to 10 carbon atoms which may optionally be substituted, or an aralkyl group containing 5 to 15 carbon atoms which may optionally be substituted.

When substituted, the substituent is not particularly restricted as long as there is no adverse effect on the reaction caused by the enzyme source but, there may be mentioned, for example, a halogen atom, a hydroxyl group, an aldehyde group, a carboxyl group, a cyano group, an amino group, a nitro group, or the like substituent.

As the methyl group having a substituent, there may be mentioned, for example, a chloromethyl group, a bromomethyl group, an iodomethyl group, a hydroxymethyl group, an aminomethyl group, a cyanomethyl group, or the like group.

As the alkyl group containing 2 to 10 carbon atoms which may optionally be substituted, there may be mentioned a substituted or unsubstituted ethyl group, n-propyl group, iso-propyl group, n-butyl group, sec-butyl group, tert-butyl group, n-pentyl group, n-hexyl group, or the like group. When the alkyl group contains a substituent, the substituent is not particularly restricted as long as the reduction reaction of the invention is not adversely affected but, there may be mentioned a halogen atom, a hydroxyl group, an amino group, a cyano group, or the like group as the substituent.

As the aralkyl group containing 5 to 15 carbon atoms which may optionally be substituted, there may be mentioned a benzyl group, an o-chlorobenzyl group, a m-bromobenzyl group, a p-fluorobenzyl group, a p-nitrobenzyl group, a p-cyanobenzyl group, a m-methoxybenzyl group, a phenethyl group, a naphthylmethyl group, a pyridylmethyl group, or the like group.

Preferred as R among the above-mentioned groups, an alkyl group containing 2 to 10 carbon atoms which may optionally be substituted, more preferred is an alkyl group containing 2 to 4 carbon atoms which may optionally be substituted, still more preferred is an ethyl group, an n-propyl group or an n-butyl group, and most preferred is an n-butyl group.

The compound represented by the above formula (1) is industrially available, or can be easily synthesized from materials which are industrially available. For example, 2-butylacrolein can be easily synthesized by stirring a mixture comprising n-hexanal, dimethylamine hydrochloride and a 37% formalin solution at 70°C for 24 hours (refer to Journal of chemical research, Synopses 7, 262-3 (1978)).

2. Optically active 2-substituted propanal derivative (product)

The product obtained in the process according to the embodiment is the optically active 2-substituted propanal derivative represented by the above formula (2). In the above formula (2), R is the same as in the formula (1), and * is an asymmetric carbon.

3. Process for producing an optically active 2-substituted propanal derivative

Next, the process for producing an optically active 2-substituted propanal derivative according to the embodiment of the invention is described.

According to the embodiment, the optically active 2-substituted propanal derivative of the above formula (2) is produced by stereoselectively reducing a carbon-carbon double bond of the 2-substituted acrolein derivative of the above formula (1) in the presence of an enzyme source having an activity of stereoselectively reducing the carbon-carbon double bond of said 2-substituted acrolein derivative (1).

4. Enzyme source

Herein, "an enzyme source" includes an enzyme having the above-mentioned reduction activity, as well as a culture of microorganisms having the above-mentioned reduction activity and a processed product thereof. "A culture of microorganisms" refers to a culture medium containing cells or cultured cells, and also a processed product thereof is included therein. "A processed product thereof" refers to, for example, a crude extract, lyophilized cells, acetone-dried cells, and a product derived from those cells by grinding. Moreover, the enzyme source mentioned above can be immobilized by a method known in the art and used as an immobilized enzyme or an immobilized cell. The immobilization can be carried out by the method known to the person skilled in the art (for example, a crosslinking method, a physical adsorption method, an inclusion method, etc.).

According to the embodiment, as the enzyme source having an activity of stereoselectively reducing the carbon-carbon double bond of the compound of the above formula (1), there may be mentioned those derived from a microorganism belonging to the genus *Candida,* the genus *Kluyveromyces*, the genus *Pichia,* the genus *Rhodotorula*, the genus *Saccharomyces,* the genus *Sporidiobolus*, the genus *Spolobolomyces,* the genus *Trigonopsis,* the genus *Zygosaccharomyces*, the genus *Achramobacter,* the genus *Acidiphilium,* the genus *Alcaligenes,* the genus *Arthrobacter,* the genus *Bacillus,* the genus *Corynebacterium,* the genus *Escherichia,* the genus *Micrococcus*, the genus *Pseudomonas,* the genus *Paenibacillus,* or the genus *Xanthomonas.*

4-1. Enzyme source (examples of an enzyme source having an activity of R-selective reduction)

Among the above-mentioned enzyme sources, as the enzyme source having an activity of R-selectively reducing the carbon-carbon double bond of the compound of the above formula (1), preferred are those derived from microorganisms such as *Candida cantarellii, Candida etchellsii, Candida kefyr, Candida musae, Candida nitrataphila, Candida sake, Candida stellata, Candida zeylanoides, Kluyveromyces lactis* var. *drosphzlarum, Pichia membranaefaciens, Pichia heedii, Rhodotorula minuta, Saccharomyces unisporus, Saccharomyces bayanus, Saccharomyces cerevisiae, Saccharomyces castellii, Saccharomyces pastorianus*, *Sporidiobolus johnsonii, Sporidiobolus salmonicolor, Spolobolomyces salmonicolor, Trigonopsis variabilis, Zygosaccharomyces bailii, Arthrobacter nicotianae, Acidiphilium cryptum, Bacillus cereus, Bacillus coagulans, Bacillus licheniformis, Bacillus pumilus, Bacillus badius, Bacillus sphaericus, Micrococcus luteus, Pseudomonas stutzeri, Pseudomonas fragi, Pseudomonas putida, Paenibacillus alvei* and *Xanthomonas* sp.

4-2. Enzyme source (examples of an enzyme source having an activity of S-selective reduction)

As the enzyme source having an activity of S-selectively reducing the carbon-carbon double bond of the compound of the above formula (1), preferred are those derived from microorganisms such as *Achromobacter xylosoxidans* subsp. *denitrificans, Alcaligenes faecalis, Alcaligenes* sp., *Arthrobacter crystallopoietes, Arthrobacter protophormise, Corynebacterium ammoniagenes*, and *Escherichia coli.*

5. Microorganism

In addition, the microorganism from which the above-mentioned reduction enzymes are derived may be a wild strain or a variant. A microorganism derived by a genetic engineering technique such as cell fusion or gene manipulation can be used as well.

Furthermore, it is also possible to use a recombinant microorganism capable of producing a reductase derived from these microorganisms. The recombinant microorganism capable of producing such enzyme can be obtained, for example, by a method comprising a step of isolating and/or purifying such enzyme and then determining a part or whole of the amino acid sequence thereof, a step of obtaining a DNA sequence coding for the enzyme based on the amino acid sequence mentioned above, a step of obtaining a recombinant microorganism by introducing the DNA mentioned above into another microorganism, and a step of obtaining the enzyme by culturing the recombinant microorganism mentioned above (refer to the process disclosed in International Publication WO98/35025).

As such recombinant microorganism mentioned above, there may be mentioned one obtained by transforming a host microorganism with a vector containing DNA coding for said reductase. As the host microorganism, *Escherichia coli* is preferred. More preferred is *Escherichia coli* HB101 (pTSYE2) transformed with a vector containing a gene of NADPH dehydrogenase derived from *Saccharomyces cerevisiae* (Old Yellow Enzyme 2) mentioned above (refer to The Journal of Biological chemistry, 268, 6097-6106 (1993)), and the like. The process for obtaining *Escherichia coli* HB101 (pTSYE2) is described in below-mentioned Example 7.

Furthermore, as the above enzyme source, it is also possible to use an oxidoreductase classified into EC 1.6.99 according to the enzyme taxonomy of International Union of Biochemistry and Molecular Biology. As the oxidoreductases classified into EC 1.6.99, there may be mentioned an enzyme classified into EC 1.6.99.1: NADPH dehydrogenase, an enzyme classified into EC 1.6.99.2: NAD(P)H dehydrogenase (quinone), an enzyme classified into EC 1.6.99.3: NADH dehydrogenase , an enzyme classified into EC 1.6.99.5: NADH dehydrogenase (quinone), and an enzyme classified into EC 1.6.99.6: NADPH dehydrogenase (quinone). Among these, NADPH dehydrogenase classified into EC 1.6.99.1 (another name: Old Yellow Enzyme) is preferred.

It has been reported that NADPH dehydrogenase is widely distributed among yeast belonging to the genus *Candida,* the genus *Kluyveromyces,* the genus *Saccharomyces,* or the genus *Schizosaccharomyces*. In particular, NADPH dehydrogenase derived from *Saccharomyces cerevisiae* (Old Yellow Enzyme 2) is preferred.

The culture medium for the microorganism which is used as an enzyme source is not particularly restricted so long as the microorganism can grow thereon. For example, a normal liquid medium containing, as a carbon source, sugar such as glucose and sucrose, alcohols such as ethanol and glycerine, fatty acids such as oleic acid and stearic acid, and esters thereof, oils such as rapeseed oil and soybean oil; as a nitrogen source, ammonium sulfate, sodium nitrate, peptone, casamino acid, corn steep liquor, bran, yeast extract, etc.; as a inorganic salt, magnesium sulfate, sodium chloride, calcium carbonate, calcium monohydrogen phosphate, potassium dihydrogen phosphate, etc.; and, as an other nutrition source, malt extract, meat extract, etc.. Culture is carried out aerobically, and usually, culture period is about 1 to 5 days, pH of the medium is 3 to 9, and culture temperature is 10 to 50°C.

6. Reduction reaction

In the embodiment, the reduction reaction of the carbon-carbon double bond in the compound represented by the above formula (1) can be carried out by adding the 2-substituted acrolein derivative of the formula (1) to serve as a substrate, the coenzyme NAD(P)H, and a cultured product derived from the above microorganism or a processed product thereof to an appropriate solvent, and stirring the mixture under pH adjustment.

The conditions of the above reduction reaction are various depending on the enzyme and the microorganism or processed product thereof to be used, concentration of the substrate, and the like. Generally, concentration of the substrate may be approximately 0.1 to 100% by weight and preferably 1 to 60% by weight, concentration of the coenzyme NAD(P)H may be 0.0001 to 100 mole% and preferably 0.0001 to 0.1 mole% relative to the concentration of the substrate, the reaction temperature may be 10 to 60°C and preferably 20 to 50°C, the pH during the reaction may be 4 to 9 and preferably 5 to 8, and the reaction period may be 1 to 120 hours and preferably 1 to 72 hours. In addition, an organic solvent can be used as a mixture with the other ingredients in the reaction. As an organic solvent, there may be mentioned, for example, toluene, ethyl acetate, n-butyl acetate, hexane, isopropanol, methanol, diisopropyl ether, acetone, dimethyl sulfoxide, and the like.

The substrate can be added at once or continuously. The reaction can be carried out batchwise or continuously.

7. Reduction reaction (example using a coenzyme regenerating system)

In the reduction process of the embodiment, it is possible to use a coenzyme NAD(P)H regenerating system, which is generally used in combination. Thereby, the amount of use of an expensive coenzyme can be substantially decreased. As a representative NAD(P)H regenerating system, there may be mentioned, for example, a process using glucose dehydrogenase and glucose.

When a transformed microorganism prepared by introducing a gene of a reductase and a gene of an enzyme (e.g., glucose dehydrogenase) capable of regenerating a coenzyme to be a part of the reductase into one and the same host microorganism, that is, a cultured product of a transformed microorganism prepared by introducing DNA coding for

the reductase according to the embodiment and a gene of an enzyme (e.g., glucose dehydrogenase) capable of regenerating a coenzyme to be a part of the reductase into one and the same host microorganism, or a processed product thereof, etc. is used to carry out the same reaction as mentioned above, the optically active 2-substituted propanal derivative can be produced at lower cost since it is not necessary to prepare separately an enzyme source required for regenerating the coenzyme.

As such transformed microorganism mentioned above, there may be mentioned one transformed with a plasmid containing both of the above-mentioned DNA coding for a reductase and DNA coding for an enzyme capable of regenerating a coenzyme to be a part of the reductase. Herein, as the enzyme capable of regenerating the coenzyme, glucose dehydrogenase is preferred, and glucose dehydrogenase derived from *Bacillus megaterium* is more preferred. And as the host microorganism, *Escherichia coli* is preferred. As such preferable transformed microorganism, *Escherichia coli* HB101 (pTSYE2G1) described in Examples below can be mentioned.

The transformed microorganism can be cultured on a liquid nutrient medium containing ordinary carbon sources, nitrogen sources, inorganic salts, organic nutrients and so forth so long as the microorganism can grow thereon.

In addition, the activity of the enzyme capable of regenerating a coenzyme in the transformed microorganism can be determined by conventional methods. For example, the activity of glucose dehydrogenase can be calculated by adding 100 mM of glucose, 2 mM of the coenzyme NADP or NAD, and the enzyme into 1M tris hydrochloride buffer (pH 8.0), subjecting the obtained mixture to reaction at 25°C for 1 minute, and measuring the rate of increase in absorbance at a wavelength of 340 nm.

When the reduction process of the invention is carried out in combination with a coenzyme regeneration system, or a cultured product of the above recombinant microorganism or the processed product thereof is used as the enzyme source, it is also possible to carry out the reaction by using, as a coenzyme, oxidized NAD(P) available at lower cost.

8. Purification of an optically active 2-substituted propanal derivative

A process for purifying the optically active 2-substituted propanal derivative produced by the reduction reaction is not particularly restricted. For example, the derivative can be purified by extracting the same with an organic solvent, for example, ethyl acetate, toluene, t-butylmethyl ether, hexane and methylene chloride, directly or after separating cells, etc. from a reaction mixture, and then subjecting the extract to dehydration, concentration, and processes such as distillation and chromatography. For the separation of cells from a reaction mixture, conventional methods such as centrifugation and filtration can be used.

9. Conversion into other derivatives

After obtaining the optically active 2-substituted propanal derivative of the above formula (2) by the above-mentioned processes, oxidation of an aldehyde group of said compound can produce an optically active 2-substituted propionate derivative. Alternatively, reduction of an aldehyde group of the compound of the formula (2) can produce an optically active 2-substituted propanol derivative.

As a process for oxidizing an aldehyde group, there may be mentioned a chemical technique using potassium permanganate ($KMnO_4$), chromic acid ($CrO_3$), nitric acid, and the like, or an enzymatic technique using an aldehyde dehydrogenase, and the like. Either technique can be used.

Furthermore, as a process for reducing an aldehyde group, there may be mentioned a chemical technique using a metal hydride such as lithium aluminum hydride ($LiAlH_4$) and sodium borohydride ($NaBH_4$), or an enzymatic technique using an aldehyde reductase, and the like. Either technique can be used.

BEST MODE FOR CARRYING OUT THE INVENTION

The following examples illustrate the present invention in further detail. These examples are, however, by no means limitative of the scope of the invention.

(Example 1) Process for producing optically active 2-methyl-1-hexanal

A liquid medium (pH 6.5) comprising glucose (5%), peptone (1%) and yeast extract (1%) was prepared to be dispensed into 500 ml Sakaguchi flasks by 50 ml fractions, and each flask was subjected to steam sterilization at 120°C for 20 minutes. A loopful of the microorganisms shown in Table 1 was respectively inoculated into each of these liquid media to be subjected to shaking culture at 30°C for 2 to 3 days. Cells were separately collected from each of these culture media by centrifugation, washed with water, added with ice-cold acetone, and then subjected to vacuum drying to prepare acetone dried cells. Each 5-mg-portion of the obtained acetone dried cells was scaled into a test tube equipped with a plug, and then suspended into 500 μl of 100 mM phosphate buffer (pH 6.5). To each of these solutions, glucose

25 mg, NAD and NADP, 0.5 mg of each, and glucose dehydrogenase (product of Amano Enzyme Inc.) 5 U were added. After each 2-mg-portion of the substrate 2-butylacrolein was dissolved in a 500-μl-portion of ethyl acetate to be added into each of the test tubes each equipped with a plug, these tubes were stirred at 30°C for 20 hours. After the reaction, each reaction mixture was centrifuged, and each of the substrate together with the product in an ethyl acetate layer was converted into trifluoroacetyl derivative thereof to be analyzed with gas chromatography (GC). Thus, the reaction conversion rates and optical purities of the products were determined. The results are shown in Table 1 (the reaction conversion is 10 to 100%).

The analysis conditions and the method for calculating the reaction conversion rate and optical purity were as follows.

[GC analysis conditions]

Capillary column: Cyclodex-β φ0.25 mm I.D. x 60 m (product of J&W Scientific Inc.)

Carrier gas: He 300 kPa

Detector: FID

Column temperature: 45°C

Detection time: 49.9 minutes for (R)-2-methyl-1-hexanal, 51.5 minutes for (S)-2-methyl-1-hexanal, and 44.6 minutes for 2-butylacrolein

$$\text{Reaction conversion rate (\%)} = \text{amount of product} / (\text{amount of substrate} + \text{amount of product}) \times 100$$

$$\text{Optical purity (\%ee)} = (A-B)/(A+B) \times 100$$

(both of A and B represent the amount of enantiomer, and the relation of A>B is satisfied)

Table 1

| Microorganism | | Reaction conversion rate (%) | Optical purity (%ee) | Configuration |
|---|---|---|---|---|
| *Candida cantarellii* | NBRC 1261 | 40.2 | 96.7 | *R* |
| *Candida etchellsii* | NBRC 1229 | 18.2 | 95.9 | *R* |
| *Candida kefyr* | NBRC 0706 | 27.9 | 97.3 | *R* |
| *Candida musae* | NBRC 1582 | 36.7 | 73.4 | *R* |
| *Candida nitratophila* | NBRC 10004 | 37.6 | 90.2 | *R* |
| *Candida sake* | CBS 5093 | 32.1 | 92.2 | *R* |
| *Candida stellata* | NBRC 0701 | 14.8 | 89.9 | *R* |
| *Candida zeylanoides* | NBRC 0738 | 27.2 | 93.1 | *R* |
| *Kluyveromyces lactis* var. *drosophilarum* | NBRC 1012 | 18.8 | 97.0 | *R* |
| *Pichia membranaefaciens* | IAM 4258 | 16.9 | 91.8 | *R* |
| *Pichia canadensis* | NBRC 0976 | 18.4 | 95.0 | *R* |
| *Pichia heedii* | NBRC 10019 | 48.8 | 92.6 | *R* |
| *Rhodotorula minuta* | NBRC 0387 | 46.7 | 95.3 | *R* |
| *Saccharomyces unisporus* | NBRC 0215 | 23.8 | 95.8 | *R* |
| *Saccharomyces bayanus* | NBRC 0213 | 20.1 | 91.4 | *R* |
| *Saccharomyces cerevisiae* | ATCC26108 | 48.2 | 92.4 | *R* |
| *Saccharomyces pastorianus* | NBRC 1265 | 35.9 | 96.9 | *R* |
| *Saccharomyces castellii* | NBRC 0285 | 27.3 | 94.3 | *R* |
| *Sporidiobolus johnsonii* | NBRC 6903 | 39.8 | 69.1 | *R* |
| *Sporidiobolus salmonicolor* | NBRC 1035 | 16.5 | 74.8 | *R* |
| *Sporobolomyces salmonicolor* | IAM 12249 | 20.2 | 84.0 | *R* |

(continued)

| Microorganism | | Reaction conversion rate (%) | Optical purity (%ee) | Configuration |
|---|---|---|---|---|
| *Trigonopsis variabilis* | NBRC 0671 | 15.1 | 81.9 | *R* |
| *Zygosaccharomyces bailii* | NBRC 0488 | 28.3 | 93.9 | *R* |

(Example 2) Process for producing optically active 2-methyl-1-hexanal

A liquid medium (pH 6.5) comprising meat extract (1%), peptone (1%), yeast extract (0.5%) and sodium chloride (1%) was prepared to be dispensed into large test tubes by 5 ml fractions, and each tube was subjected to steam sterilization at 120°C for 20 minutes. A loopful of the microorganisms shown in Table 2 was respectively inoculated into each of these liquid media to be subjected to shaking culture at 30°C for 2 to 3 days. Cells were collected from each of these culture media by centrifugation. Each cell portion was washed with a 1-ml-portion of 100 mM phosphate buffer (pH 6.5), suspended into a 500-μl-portion of the same buffer, and then put into a test tube equipped with a plug. To each of these solutions, glucose 25 mg, NAD and NADP, 0.5 mg of each, and glucose dehydrogenase (product of Amano Enzyme Inc.) 5 U were added. After each 2-mg-portion of the substrate 2-butylacrolein was dissolved in a 500-μl-portion of ethyl acetate to be added into each of the test tubes each equipped with a plug, these tubes were stirred at 30 °C for 20 hours. After the reaction, each reaction mixture was centrifuged, and the amounts of substrate and product in an ethyl acetate layer were analyzed in the same manner as in Example 1 to determine the reaction conversion rate and optical purity of the product. The results are shown in Table 2 (the reaction conversion is 10 to 100%).

Table 2

| Microorganism | | Reaction conversion rate (%) | Optical purity (%ee) | Configuration |
|---|---|---|---|---|
| *Arthrobacter nicotianae* | NBRC 14234 | 45.2 | 42.9 | *R* |
| *Acidiphilium cryptum* | NBRC 14242 | 44.8 | 45.7 | *R* |
| *Bacillus cereus* | NBRC 3466 | 21.9 | 84.3 | *R* |
| *Bacillus coagulans* | NBRC 3886 | 19.0 | 95.7 | *R* |
| *Bacillus licheniformis* | IAM 11054 | 16.2 | 83.1 | *R* |
| *Bacillus pumilus* | NBRC 12086 | 18.3 | 94.7 | *R* |
| *Bacillus badius* | ATCC 14574 | 29.1 | 88.7 | *R* |
| *Bacillus sphaericus* | NBRC 3525 | 73.3 | 90.7 | *R* |
| *Micrococcus luteus* | NBRC 13867 | 47.8 | 66.6 | *R* |
| *Pseudomonas stutzeri* | NBRC 13596 | 30.2 | 55.4 | *R* |
| *Pseudomonas fragi* | NBRC 3458 | 47.2 | 65.0 | *R* |
| *Pseudomonas putida* | NBRC 14164 | 24.7 | 13.3 | *R* |
| *Paenibacillus alvei* | NBRC 3343 | 35.7 | 89.7 | *R* |
| *Xanthomonas* sp. | NBRC 3084 | 65.0 | 86.1 | *R* |
| *Xanthomonas* sp. | NBRC 3085 | 52.2 | 86.0 | *R* |
| *Achromobacter xylosoxidans* subsp. *denitrificans* | NBRC 15125 | 11.5 | 33.5 | *S* |
| *Alcaligenes faecalis* | NERC 13111 | 15.7 | 63.2 | *S* |
| *Alcaligenes* sp. | NBRC 14130 | 10.5 | 29.9 | *S* |
| Arthrobacter *crystallopoietes* | NBRC 14235 | 12.9 | 54.0 | *S* |
| *Arthrobacter protophormiae* | NBRC 12128 | 16.8 | 25.2 | *S* |
| *Corynebacterium ammoniagenes* | NBRC 12072 | 14.4 | 65.0 | *S* |
| *Escherichia coli* | ATCC 11303 | 55.1 | 78.3 | *S* |

(Example 3) Process for producing optically active 2-methyl-1-alkylpropanal

Each 5-mg-portion of the acetone dried cells prepared in Example 1 or the cells separated from the culture medium prepared in Example 2 was suspended into a 500-μl-portion of 100 mM phosphate buffer (pH 6.5) in a test tube equipped with a plug. To each of these solutions, glucose 25 mg, NAD and NADP, 0.5 mg of each, glucose dehydrogenase (product of Amano Enzyme Inc.) 5 U were added. After each 2-mg-portion of the substrates shown in Table 3 was dissolved in a 500-μl-portion of ethyl acetate to be added into a test tube equipped with a plug, these tubes were stirred at 30°C for 20 hours. After the reaction, each reaction mixture was centrifuged, and the amounts of substrate and product in an ethyl acetate layer were analyzed with gas chromatography (GC) to determine the reaction conversion rate and optical purity of the product. The results are shown in Table 3.

The analysis conditions were as follows.
[GC analysis conditions (2-methylbutanal: trifluoroacetyl derivatization of the substrates and products)]
Capillary column: Cyclodex-β φ0.25 mm I.D.x 60 m (product of J&W Scientific Inc.)
Carrier gas: He 300 kPa
Detector: FID
Column temperature: 40°C
Detection time: 16.7 minutes for (R)-2-methyl-1-butanal, 17.0 minutes for (S)-2-methyl-1-butanal, 15.0 minutes for 2-ethylacrolein
[GC analysis conditions (2-methyloctanal)]
Capillary column: TC-WAX φ0.25 mm I.D. x 15 m (product of GL Sciences Inc.)
Carrier gas: He 80 kPa
Detector: FID
Column temperature: 80°C
Detection time: 2.8 minutes for 2-methyl-1-octanal, 3.6 minutes for 2-hexylacrolein

Table 3

| Microorganism | | 2-ethylacrolein | | | 2-hexylacrolein | | |
|---|---|---|---|---|---|---|---|
| | | Reaction conversion rate (%) | Optical purity (%ee) | Configuration | Reaction conversion rate (%) | Optical purity (%ee) | Configuration |
| *Candida cantarellii* | NBRC 1261 | 40.8 | 93.5 | *R* | 83.7 | - | - |
| *Candida etchellsii* | NBRC 1229 | 13.3 | 89.7 | *R* | 26.2 | - | - |
| *Candida kefyr* | NBRC 0706 | 25.2 | 93.2 | *R* | 39.5 | - | - |
| *Kluyveromyces lactis* var. *drosophilarum* | NBRC 1012 | 17.6 | 93.0 | *R* | 25.6 | - | - |
| *Pichia canadensis* | NBRC 0976 | 15.4 | 91.4 | *R* | 15.7 | - | - |
| *Rhodotorula minuta* | NBRC 0387 | 32.7 | 92.7 | *R* | 56.0 | - | - |
| *Saccharomyces unisporus* | NBRC 0215 | 19.2 | 90.7 | *R* | 31.7 | - | - |
| *Saccharomyces cerevisiae* | ATCC 26108 | 48.2 | 92.4 | *R* | 52.8 | - | - |
| *Saccharomyces pastorianus* | NBRC 1265 | 28.7 | 94.1 | *R* | 44.4 | - | - |
| *Bacillus coagulans* | NBRC 3885 | 12.3 | 65.9 | *R* | 6.7 | - | - |
| *Bacillus pumilus* | NBRC 12086 | 49.8 | 97.8 | *R* | 11.9 | - | - |
| *Bacillus baclius* | ATCC 14574 | 13.2 | 85.6 | *R* | 5.8 | - | - |

(continued)

| Microorganism | | 2-ethylacrolein | | | 2-hexylacrolein | | |
|---|---|---|---|---|---|---|---|
| | | Reaction conversion rate (%) | Optical purity (%ee) | Configuration | Reaction conversion rate (%) | Optical purity (%ee) | Configuration |
| *Bacillus sphaericus* | NBRC 3525 | 51.2 | 79.5 | *R* | 19.0 | - | - |
| *Paenibacillus alvei* | NBRC 3343 | 10.0 | 80.4 | *R* | 91.6 | | |
| *Xanthomonas* sp. | NBRC 3084 | 16.4 | 89.0 | *R* | 22.1 | - | - |
| *Xanthomonas* sp. | NBRC 3085 | 14.3 | 90.0 | *R* | 18.0 | - | - |
| *Alcaligenes faecalis* | NBRC 13111 | 0.7 | 40.5 | *S* | 0.5 | - | - |
| *Escherichia coli* | ATCC 11303 | 18.8 | 57.0 | *S* | 18.0 | - | - |

(Example 4) Process for producing optically active 2-methyl-3-phenylpropanal

Each 5-mg-portion of acetone dried cells prepared in Example 1 or the cells separated from the culture medium prepared in Example 2 was suspended into a 500-μl-portion of 100 mM phosphate buffer (pH 6.5) in a test tube equipped with a plug. To each of these solutions, glucose 25 mg, NAD and NADP, 0.5 mg of each, and glucose dehydrogenase (product of Amano Enzyme Inc.) 5 U were added. After each 2-mg-portion of the substrate 2-benzylacrolein was dissolved in a 500-μl-portion of ethyl acetate to be added into each of the test tubes each equipped with a plug, these tubes were stirred at 30°C for 20 hours. After the reaction, each reaction mixture was centrifuged, and each of the substrate together with the product in an ethyl acetate layer was converted into trifluoroacetyl derivertive thereof to be analyzed with gas chromatography (GC). Thus, the reaction conversion rates and optical purities of the products were determined. The results are shown in Table 4.

The analysis conditions were as follows.

[GC analysis conditions]

Capillary column: Cyclodex-β φ0.25 mm I.D. x 60 m (product of J&W Scientific Inc.)

Carrier gas: He 300 kPa

Detector: FID

Column temperature: 80°C

Detection time: 113.9 minutes for (R)-2-methyl-3-phenylpropanal, 116.9 minutes for (S)-2-methyl-3-phenylpropanal, 96.2 minutes for 2-benzylacrolein

Table 4

| Microorganism | | Reaction conversion rate (%) | Optical purity (%ee) | Configuration |
|---|---|---|---|---|
| *Candida cantarellii* | NBRC 1261 | 63.4 | 86.4 | *R* |
| *Candida etchellsii* | NBRC 1229 | 22.2 | 43.0 | *R* |
| *Candida kefyr* | NBRC 0706 | 33.7 | 85.6 | *R* |
| *Kluyveromyces lactis* var. *drosophilarum* | NBRC 1012 | 25.1 | 42.0 | *R* |
| *Pichia canadensis* | NBRC 0976 | 18.0 | 68.0 | *R* |
| *Rhodotorula minuta* | NBRC 0387 | 61.1 | 83.2 | *R* |
| *Saccharomyces unisporus* | NBRC 0215 | 32.9 | 33.8 | *R* |
| *Saccharomyces cerevisiae* | ATCC 26108 | 61.6 | 94.2 | *R* |
| *Saccharomyces pastorianus* | NBRC 1265 | 44.5 | 78.0 | *R* |

(continued)

| Microorganism | | Reaction conversion rate (%) | Optical purity (%ee) | Configuration |
|---|---|---|---|---|
| *Bacillus coagulans* | NBRC 3886 | 12.3 | 22.0 | *R* |
| *Bacillus pumilus* | NBRC 12086 | 32.8 | 14.8 | *R* |
| *Bacillus badius* | ATCC 14574 | 10.3 | 55.0 | *R* |
| *Bacillus sphaericus* | NBRC 3525 | 39.1 | 67.0 | *R* |
| *Paenibacillus alvei* | NBRC 3343 | 9.1 | 55.9 | *R* |
| *Xanthomonas* sp. | NBRC 3084 | 7.4 | 65.3 | *R* |
| *Xanthomonas* sp. | NBRC 3085 | 7.4 | 55.2 | *R* |
| *Alcaligenes faecalis* | NBRC 13111 | 2.7 | 81.2 | *S* |
| *Escherichia coli* | ATCC 11303 | 57.4 | 70.2 | *S* |

(Example 5) Cloning of NADPH dehydrogenase gene derived *from Saccharomyces cerevisiae* S288C (ATCC26108) strain (Old Yellow Enzyme 2 gene)

An expression vector was prepared by the method mentioned below for expressing an NADPH dehydrogenase derived from *Saccharomyces cerevisiae* S288C (ATCC26108) strain (Old Yellow Enzyme 2, hereinafter, referred to as "OYE2") in *Escherichia coli* (refer to Appl. Environ. Microbiol., 69, 933, (2003)).

First, a DNA fragment containing OYE2 gene was amplified by a first-step PCR using a chromosomal DNA of *Saccharomyces cerevisiae* S288C (ATCC26108) strain as a template. Then, by a second-step PCR using the DNA fragment obtained by the first-step PCR as a template, a double-stranded DNA was obtained in which a SacI site is added to the initiation codon site of OYE2 gene and a new termination codon and a SalI site are added to just after the termination codon. Details are shown below.

A primer 1: 5'-cggtccagatatagaataaatcatcatattaag-3'(SEQ ID NO:1 in the sequence listing) and a primer 2: 5'-gaaatggtgctacaaagtacggttaacac-3'(SEQ ID NO:2 in the sequence listing) were synthesized. 50 μl of an ExTaq buffer containing each 50 pmol of these two species of primers (primers 1 and 2), 200 ng of a chromosomal DNA of *Saccharomyces cerevisiae* S288C (ATCC26108) strain, each 10 nmol of dNTP, and 2.5 U of ExTaq (product of Takara Shuzo Co., Ltd.) was prepared. A PCR reaction was carried out under the conditions of thermal denaturation (97°C, 0.5 minute), annealing (55°C, 1 minute), and elongation (72°C, 1 minute). After cooling the buffer to 4°C, amplification of the DNA fragment was confirmed by agarose gel electrophoresis, and the amplified fragment was collected from gel. The chromosomal DNA was prepared by usual DNA isolation method, e.g., a potassium acetate method, etc. Then, a primer 3: 5'-atcgagctctaaggaggttaacaatgccatttgttaaggac-3' (SEQ ID NO:3 in the sequence listing) resulting from addition of the *Escherichia coli*-derived Shine-Dalgarno sequence (SD sequence, 9 nucleotides) at upstream of the initiation codon of OYE2 gene and a SacI recognition site just before thereof, and a primer 4: 5'-acgcgtcgacttattaatttttgtcccaaccg-3' (SEQ ID NO:4 in the sequence listing) resulting from addition of a new termination codon and a SalI recognition site just after the termination codon were synthesized. 50 μl of an ExTaq buffer containing each 50 pmol of these two species of primers (primers 3 and 4), 200 ng of the DNA fragment amplified by the PCR reaction mentioned above, each 10 nmol of dNTP, and 2.5 U of ExTaq (product of Takara Shuzo Co., Ltd.) was prepared to carry out a PCR reaction under the same conditions as mentioned above. As a result, a double-stranded DNA was obtained in which the *Escherichia coli*-derived Shaine-Dalgarno sequence (SD sequence) is added at a site 5 bases upstream of the initiation codon of OYE2 gene and a SacI recognition site is added just before that sequence and, further, a new termination codon and a SalI recognition site are added just after the termination codon. This double-stranded DNA was digested with SacI and SalI, and the digest was inserted into the SacI and SalI sites at downstream of lac promoter of a plasmid pUCT resulting from conversion, in an NdeI site of a plasmid pUCNT (obtainable by the person skilled in the art by the method described in International Publication WO94/03613), of G into T in order to construct a recombinant vector pTSYE2.

(Example 6) Construction of an expression vector further comprising a glucose dehydrogenase gene

Using a primer 5: 5'-acgcgtcgactaaggaggttaacaatgtataa agatttagaagg-3' (SEQ ID NO:5 in the sequence listing) and a primer 6: 5'-gcgctgcagttatccgcgtcctgcttgga-3'(SEQ ID NO:6 in the sequence listing), and using a plasmid pGDK1 (refer to Eur. J. Biochem., 186, 389 (1989)) as a template, PCR was carried out to obtain a double-stranded DNA in which the *Escherichia* coli-derived Shaine-Dalgarno sequence (SD sequence) is added at a site 5 bases upstream of the initiation codon of a glucose dehydrogenase (hereinafter referred to as "GDH") gene derived from the *Bacillus megaterium* IAM 1030 strain, a SalI recognition site is added just before that sequence and, further, a new termination

codon and a PstI recognition site is added just after the termination codon. This double-stranded DNA obtained was digested with SalI and PstI. The digest was inserted between a SalI recognition site and PstI recognition site of the above recombinant vector pTSYE2 to construct a recombinant vector pTSYE2G1. The process for producing pTSYE2G1 and the construction thereof are shown in Fig.1.

(Example 7) Production of a transformant

Using the recombinant vector pTSYE2 constructed in Example 5, *Escherichia coli* HB101 (product of Takara Shuzo Co., Ltd.) was transformed to obtain a strain transformant *Escherichia coli* HB101 (pTSYE2). In the same manner, using the recombinant vector pTSYE2G1 constructed in Example 6, *Escherichia coli* HB101 (product of Takara Shuzo Co., Ltd.) was transformed to obtain a transformant *Escherichia coli* HB101 (pTSYE2G1).

(Example 8) Gene expression in a transformant

Two species of transformants obtained in Example 7 and a transformant *Escherichia coli* HB101 (pUCT) introduced with a vector plasmid pUCT (refer to Example 5) alone were respectively inoculated into a 2xYT liquid medium (bactotriptone 1.6%, bactoyeast extract 1.0%, sodium chloride 0.5%, pH 7.0) containing 100 μg/ml of ampicillin, and subjected to shaking culture at 37°C for 24 hours. From the culture media, cells were collected by centrifugation, suspended into 100 mM phosphate buffer (pH 6.5), and subjected to ultrasonic disruption using an UH-50 type ultrasonic homogenizer (product of SMT Co., Ltd). Then, cell residues were removed by centrifugation to obtain a cell-free extraction. The OYE2 activity and GDH activity of this cell-free extraction were determined and shown in Table 5.

In both two species of transformants obtained in Example 7, expression of OYE2 activity was observed. Moreover, in a GDH gene-containing transformant *Escherichia coli* HB101 (pTSYE2G1), expression of GDH activity was also observed.

Additionally, the OYE2 activity was calculated by adding 1 mM of the substrate 2-cyclohexenone, 0.2 mM of coenzyme NADPH and a crude enzyme solution to 100 mM phosphate buffer (pH 6.5), subjecting the mixture to reaction at 30°C for 1 minute, and measuring the rate of decrease in absorbance at a wavelength of 340 nm. The enzyme activity for oxidizing 1 μmol of NADPH to $NADP^+$ per minute under these reaction conditions was defined as 1 unit.

The GDH activity was calculated by adding 0.1 M of glucose, 2 mM of coenzyme NADP, and a crude enzyme solution to 1 M tris hydrochloride buffer (pH 8.0), subjecting the mixture to reaction at 25°C for 1 minute, and measuring the rate of increase in absorbance at a wavelength of 340 nm. The enzyme activity for reducing 1 μmol of NADP to NADPH per minute under these reaction conditions was defined as 1 unit.

Table 5

| Strain | OYE2 activity (U/ml) | GDH activity (U/ml) |
|---|---|---|
| *E. coli* HB101(pUCT) | 0.00 | 0.0 |
| *E. coli* HB101(pTSYE2) | 0.30 | 0.0 |
| *E. coli* HB101(pTSYE2G1) | 1.20 | 82.0 |

(Example 9) Process for producing (R)-2-methyl-1-alkylpropanal using a transformant

The transformant *Escherichia coli* HB101 (pTSYE2) obtained in Example 7 was inoculated to 50 ml of a 2xYT medium (bactotriptone 1.6%, bactoyeast extract 1.0%, sodium chloride 0.5%, pH 7.0) sterilized in a 500 ml Sakaguchi flask, and subjected to shaking culture at 30°C for 2 days. The culture medium obtained was concentrated and subjected to ultrasonic disruption using an UH-50 type ultrasonic homogenizer (product of SMT Co., Ltd). Then, cell residues were removed by centrifugation to obtain a cell-free extraction. In 450 μl of 100 mM phosphate buffer (pH 6.5), this cell-free extraction 50 μl, glucose 25 mg, NAD and NADP, 0.5 mg of each, and glucose dehydrogenase (product of Amano Enzyme Inc.) 5 U were dissolved, and the solution was put into a test tube equipped with a plug. After each 2-mg-portion of the substrates shown in Table 6 was dissolved in a 500-μl-portion of ethyl acetate to be added into each of the test tubes each equipped with a plug, these tubes were stirred at 30°C for 2 hours. After the reaction, each reaction mixture was centrifuged and the amounts of substrate and product in an ethyl acetate layer were analyzed in the same manner as in Examples 1 and 3 to determine the reaction conversion rate and optical purity of the product. The results are shown in Table 6.

Table 6

| Substrate | Reaction conversion rate | Optical purity (%ee) |
|---|---|---|
| 2-ethylacrolein | 96.7 | 96.0 |
| 2-butylacrolein | 99.7 | 94.5 |
| 2-hexylacrolein | 98.6 | - |

(Example 10) Process for producing (R)-2-methyl-3-phenylpropanal using a transformant

50 μl of a cell-free extraction of the transformant *Escherichia coli* HB101 (pTSYE2) prepared in the same manner as in Example 9, glucose 25 mg, NAD and NADP, 0.5 mg of each, and glucose dehydrogenase (product of Amano Enzyme Inc.) 5 U were dissolved in 450 μl of 100 mM phosphate buffer (pH 6.5), and put into a test tube equipped with a plug. Then, 2 mg of the substrate 2-benzylacrolein was dissolved in 500 μl of ethyl acetate and added into the test tube equipped with a plug, and then stirred at 30°C for 2 hours. After the reaction, the reaction mixture was centrifuged and the amount of substrate and product in an ethyl acetate layer was analyzed in the same manner as in Example 4. As a result, (R)-2-methyl-3-phenylpropanal with the optical purity of 94.7%ee was obtained at the reaction conversion rate of 99.3%.

(Example 11) Process for producing (R)-2-methyl-1-hexanal using a transformant

The transformant *Escherichia coli* HB101 (pTSYE2G1) obtained in Example 7 was inoculated to 50 ml of a 2xYT medium (bactotriptone 1.6%, bactoyeast extract 1.0%, sodium chloride 0.5%, pH 7.0) sterilized in a 500 ml Sakaguchi flask to be subjected to shaking culture at 30°C for 2 days. The culture medium obtained was concentrated and subjected to ultrasonic disruption using an UH-50 type ultrasonic homogenizer (product of SMT Co., Ltd). Then, cell residues were removed by centrifugation to obtain a cell-free extraction. 30 ml of the above cell-free extraction, 195 ml of 100 mM phosphate buffer (pH 6.5), 25 ml of 55% glucose solution, and 25 mg of NADP were added to a 500 ml 4-necked flask. Then, 1 g of the substrate 2-butylacrolein was dissolved in 100 ml of hexane, and added dropwise into the reaction system for 25 minutes. Under pH adjustment, the obtained liquid was stirred at 30°C for 1 hour, and the amount of substrate and product in a hexane layer was analyzed in the same manner as in Example 1. Then, the reaction mixture was separated and hexane was distilled off under reduced pressure to obtain (R)-2-methyl-1-hexanal with the optical purity of 96.3%ee in 80% yield.

(Example 12) Process for producing 5-hydroxy-2-methyl-1-pentanal using a transformant

500 μl of a culture medium of the transformant *Escherichia coli* HB101 (pTSYE2G1) prepared in the same manner as in Example 11, 30 μl of 55% glucose solution and 0.5 mg of NADP were mixed with 100 μl of 100 mM phosphate buffer (pH 6.5), and the mixture was put into a test tube equipped with a plug. Then, 10 mg of the substrate 2-(3-hydroxypropyl)acrolein was added thereto and the mixture was stirred for 2 hours. After the reaction, the reaction mixture was extracted with ethyl acetate, and the amount of substrate and product in an ethyl acetate layer was analyzed with GC under the below-mentioned analysis conditions. As a result, 5-hydroxy-2-methyl-1-pentanal was obtained at the reaction conversion rate of 96.9%.

The analysis conditions were as follows. [GC analysis conditions]
Capillary column: β-DEX225 φ0.25 mm I.D. x 30 m (product of SUPELCO Co., Ltd)
Carrier gas: He 100 kPa
Detector: FID
Column temperature: 120°C
Detection time: 7.1 minutes for 5-hydroxy-2-methyl-1-pentanal, 14.8 minutes for 2-(3-hydroxypropyl)acrolein

(Example 13) Process for producing (R)-5-benzyloxy-2-methyl-1-pentanal using a transformant

500 μl of a culture medium of the transformant *Escherichia coli* HB101 (pTSYE2G1) prepared in the same manner as in Example 11, 30 μl of 55% glucose solution and 0.5 mg of NADP were mixed with 100 μl of 100 mM phosphate buffer (pH 6.5), and put into a test tube equipped with a plug. Then, 10 mg of the substrate 2-(3-benzyloxypropyl) acrolein was added thereto and the mixture was stirred for 2 hours. After the reaction, the reaction mixture was extracted with ethyl acetate, and the amounts of substrate and product in an ethyl acetate layer were analyzed with GC under the below-mentioned analysis conditions. As a result, 5-benzyloxy-2-methyl-1-pentanal was obtained at the reaction conversion rate of 99.1%. Furthermore, as a result of reducing 5-benzyloxy-2-methyl-1-pentanal with sodium borohydride

($NaBH_4$) and HPLC analysis under the below-mentioned analysis conditions, the optical purity was 90.0%ee(R).

The analysis conditions were as follows. [GC analysis conditions]
Capillary column: TC-WAX φ0.25 mm I.D. x 15 m (product of GL Sciences Inc.)
Carrier gas: He 80 kPa
Detector: FID
Column temperature: 165°C
Detection time: 11.1 minutes for 5-benzyloxy-2-methyl-1-pentanal, 13.2 minutes for 2-(3-benzyloxypropyl)acrolein
[HPLC analysis conditions]
Chiral column: OB-H φ4.6 mm I.D. x 250 mm (product of Daicel Chemical Industries, Ltd.)
Eluant: hexane /2-propanol 95/5
Detector: UV 254 nm
Column temperature: 25°C
Detection time: 12.2 minutes for (S)-5-benzyloxy-2-methyl-1-pentanol, 14.3 minutes for (R)-5-benzyloxy-2-methyl-1-pentanol

(Reference Example 1) Process for producing (R)-2-methyl-1-hexanoic acid

5 U of aldehyde dehydrogenase (product of Sigma-Aldrich Corp.) and 13 mg of NAD were dissolved in 500 μl of 100 mM phosphate buffer (pH 8.0), and put into a test tube equipped with a plug. After 2 mg of (R)-2-methyl-1-hexanal obtained in Example 11 was added thereto, the mixture was stirred at 30°C for 20 hours. After the reaction, 6 N hydrochloric acid solution was added for acidizing the reaction mixture, and 1 ml of ethyl acetate was added for extracting the substrate and product, which were analyzed with GC under the analysis conditions mentioned below. As a result, (R)-2-methyl-1-hexanoic acid with 96.5 %ee was obtained at the reaction conversion rate of 99.1%.

The analysis conditions were as follows. [GC analysis conditions]
Capillary column: G-PN ϕ 0.25 mm I.D. x 30 m (product of Tokyo Chemical Industry Co., Ltd.)
Carrier gas: He 150 kPa
Detector: FID
Column temperature: 40°C
Detection time: 22.0 minutes for (R)-2-methylhexanoic acid, 23.4 minutes for (S)-2-methylhexanoic acid, 15.1 minutes for 2-methyl-1-hexanal

## Claims

1. A process for producing an optically active 2-substituted propanal derivative represented by the general formula (2):

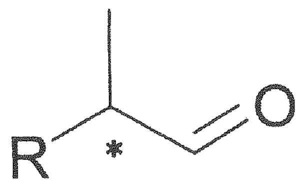

(2)

(in the formula, R is a methyl group having a substituent, an alkyl group containing 2 to 10 carbon atoms which may optionally be substituted, or an aralkyl group containing 5 to 15 carbon atoms which may optionally be substituted, and * is an asymmetric carbon)
which comprises reacting a 2-substituted acrolein derivative represented by the general formula (1):

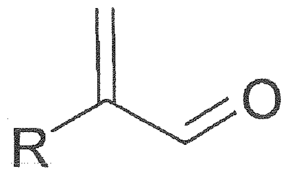

(1)

(in the formula, R is as mentioned above) with an enzyme source capable of stereoselectively reducing the carbon-carbon double bond of the 2-substituted acrolein derivative.

2. The process according to Claim 1,
wherein R is an alkyl group containing 2 to 10 carbon atoms which may optionally be substituted.

3. The process according to Claim 1,
wherein R is an alkyl group containing 2 to 4 carbon atoms which may optionally be substituted.

4. The process according to any one of Claims 1 to 3,
which comprises using, as the enzyme source capable of stereoselectively reducing the carbon-carbon double bond, an enzyme source derived from a microorganism belonging to the genus *Candida,* the genus *Kluyveromyces,* the genus *Pichia,* the genus *Rhodotorula,* the genus *Saccharomyces,* the genus *Sporidiobolus,* the genus *Spolobolomyces,* the genus *Trigonopsis,* the genus *Zygosaccharomyces,* the genus *Achromobacter,* the genus *Acidiphilium,* the genus *Alcaligenes,* the genus *Arthrobacter*, the genus *Bacillus,* the genus *Corynebacterium,* the genus *Escherichia,* the genus *Micrococcus,* the genus *Pseudomonas,* the genus *Paenibacillus,* or the genus *Xanthomonas.*

5. The process according to any one of Claims 1 to 3,
which comprises using, as the enzyme source capable of stereoselectively reducing the carbon-carbon double bond, an enzyme source derived from a microorganism belonging to the genus *Candida,* the genus *Kluyveromyces,* the genus *Pichia,* the genus *Rhodotorula*, the genus *Saccharomyces,* the genus *Sporidiobolus*, the genus *Spolobolomyces*, the genus *Trigonopsis,* the genus *Zygosaccharomyces,* the genus *Acidiphilium,* the genus *Arthrobacter,* the genus *Bacillus,* the genus *Micrococcus,* the genus *Pseudomonas,* the genus *Paenibacillus,* or the genus *Xanthomonas,* and capable of R-selective reduction of the carbon-carbon double bond of the compound represented by the above formula (1).

6. The process according to Claim 5,

wherein said enzyme source capable of R-selective reduction is an enzyme source derived from one or more microorganism(s) selected from the group consisting of *Candida cantarellii, Candida etchellsii, Candida kefyr, Candida musae, Candida nitratophila, Candida sake, Candida stellata, Candida zeylanoides, Kluyveromyces lactis* var. *drosphilarum, Pichia membranaefaciens, Pichia heedii, Rhodotorula minuta, Saccharomyces unisporus, Saccharomyces bayanus, Saccharomyces cerevisiae, Saccharomyces castellii, Saccharomyces pastorianus, Sporidiobolus johnsonii, Sporidiobolus salmonicolor, Spolobolomyces salmonicolor, Trigonopsis variabilis, 2ygosaccharomyces bailii, Arthrobacter nicotianae, Acidiphilium cryptum, Bacillus cereus, Bacillus coagulans, Bacillus licheniformis, Bacillus pumilus, Bacillus badius, Bacillus sphaericus, Micrococcus luteus, Pseudomonas stutzeri, Pseudomonas fragi, Pseudomonas putida, Paenibacillus alvei*, and *Xanthomonas* sp.

**7.** The process according to Claim 5,
wherein said enzyme source capable of R-selective reduction is a cultured product of a transformed microorganism transformed with a vector containing a gene of NADPH dehydrogenase derived from *Saccharomyces cerevisiae* (Old Yellow Enzyme 2) .

**8.** The process according to any one of Claims 1 to 3,
which comprises using, as the enzyme source capable of stereoselectively reducing the carbon-carbon double bond, an enzyme source derived from a microorganism belonging to the genus *Achromobacter,* the genus *Alcaligenes,* the genus *Arthrobacter,* the genus *Corynebacterium,* or the genus *Escherichia,* and capable of S-selective reduction of the carbon-carbon double bond of the compound represented by the above formula (1).

**9.** The process according to Claim 8,
wherein said enzyme source capable of S-selective reduction is an enzyme source derived from one or more microorganism(s) selected from the group consisting of *Achromobacter xylosoxidans* subsp. *denitrificans, Alcaligenes faecalis, Alcaligenes* sp., *Arthrobacter crystallopoietes, Arthrobacter protophormise, Corynebacterium ammoniagenes,* and *Escherichia coli.*

**10.** The process according to any one of Claims 1 to 3,
wherein an oxidoreductase classified into Enzyme Commission Number 1.6.99 is used as the enzyme source capable of stereoselectively reducing the carbon-carbon double bond.

**11.** The process according to Claim 10,
wherein NADPH dehydrogenase classified into EC 1.6.99.1 is used as the oxidoreductase classified into Enzyme Commission Number 1.6.99, and
an R form of the compound represented by the above formula (2) is produced.

**12.** The process according to Claim 11,
wherein said NADPH dehydrogenase is NADPH dehydrogenase derived from *Saccharomyces cerevisiae* (Old Yellow Enzyme 2).

Fig. 1

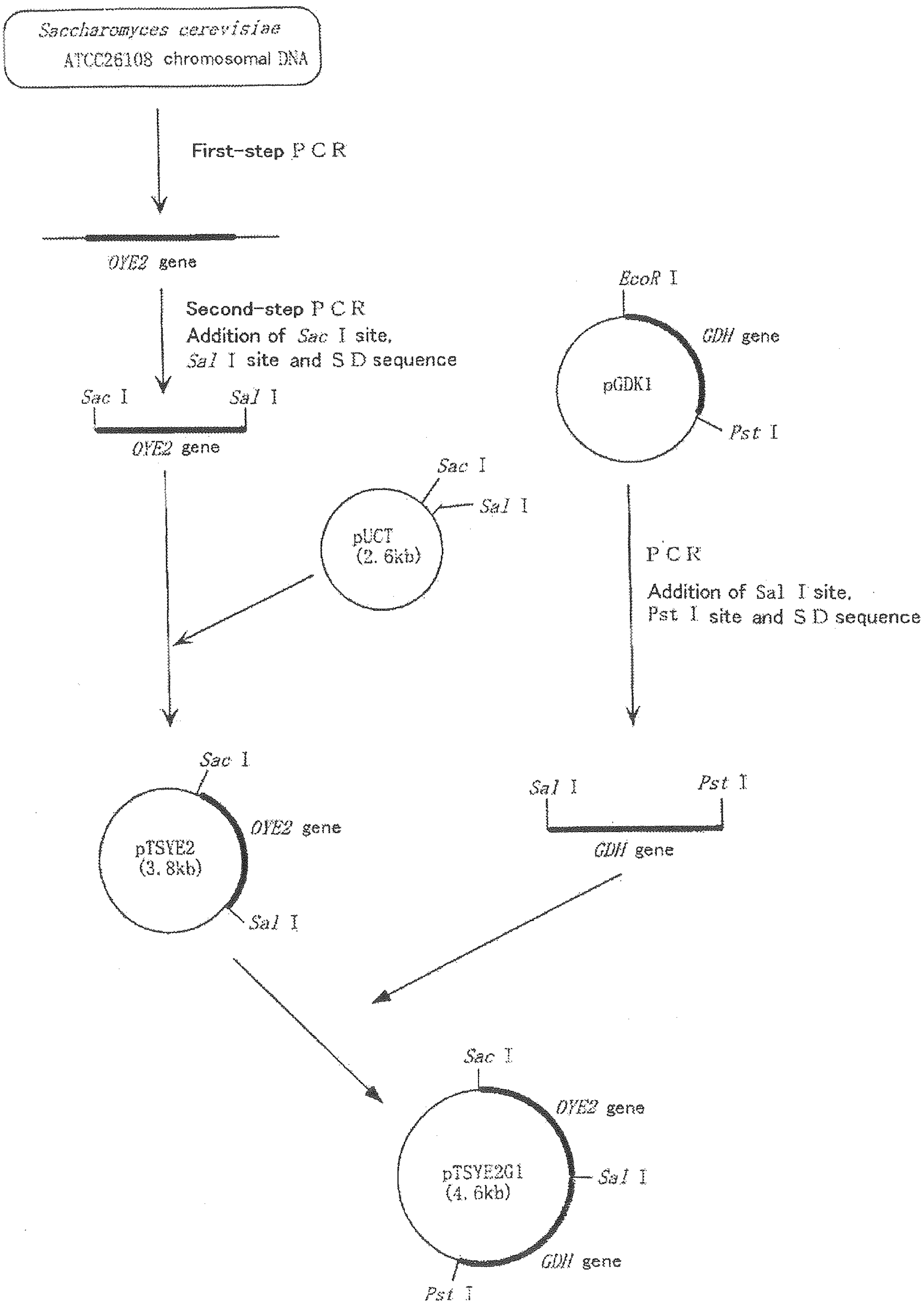
Saccharomyces cerevisiae
ATCC26108 chromosomal DNA
First-step P C R
OYE2 gene
Second-step P C R
Addition of Sac I site,
Sal I site and S D sequence
Sac I
Sal I
OYE2 gene
Sac I
Sal I
pUCT
(2. 6kb)
EcoR I
GDH gene
pGDK1
Pst I
P C R
Addition of Sal I site,
Pst I site and S D sequence
Sac I
OYE2 gene
pTSYE2
(3. 8kb)
Sal I
Sal I
Pst I
GDH gene
Sac I
OYE2 gene
pTSYE2G1
(4. 6kb)
Sal I
GDH gene
Pst I

**INTERNATIONAL SEARCH REPORT**

International application No.
PCT/JP2006/310715

A. CLASSIFICATION OF SUBJECT MATTER
**C12P7/24**(2006.01), *C12N15/09*(2006.01)

According to International Patent Classification (IPC) or to both national classification and IPC

B. FIELDS SEARCHED

Minimum documentation searched (classification system followed by classification symbols)
**C12P7/24**(2006.01), *C12N15/09*(2006.01)

Documentation searched other than minimum documentation to the extent that such documents are included in the fields searched

Electronic data base consulted during the international search (name of data base and, where practicable, search terms used)
CA/REGISTRY/BIOSIS/CASREACT/WPIDS(STN)

C. DOCUMENTS CONSIDERED TO BE RELEVANT

| Category* | Citation of document, with indication, where appropriate, of the relevant passages | Relevant to claim No. |
|---|---|---|
| A | Desrut, M.; Kergomard, A.; Renard, M.F.; Veschambre, H., Microbiological reduction of α,β-ethylenic aldehydes by Beauveria sulfurescens, Tetrahedron (1981), 37(22), 3825-9 | 1-12 |
| A | WO 2004/083166 A1 (Kaneka Corp.), 30 September, 2004 (30.09.04), Claims & EP 1604975 A | 1-12 |
| A | WO 01/94337 A1 (Kaneka Corp.), 13 December, 2001 (13.12.01), Claims & EP 1288213 A | 1-12 |

☒ Further documents are listed in the continuation of Box C.

☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 July, 2006 (04.07.06) | 11 July, 2006 (11.07.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.
PCT/JP2006/310715

C (Continuation). DOCUMENTS CONSIDERED TO BE RELEVANT

| Category* | Citation of document, with indication, where appropriate, of the relevant passages | Relevant to claim No. |
|---|---|---|
| A | JP 11-103878 A (Mitsubishi Chemical Corp.), 20 April, 1999 (20.04.99), Full text (Family: none) | 1-12 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005158393 A
- WO 9835025 A
- WO 9403613 A


### Non-patent literature cited in the description


- *New Journal of Chemistry,* 2000, vol. 24 (12), 973-975
- *Tetrahedron,* 1981, vol. 37 (22), 3825-3829
- *Journal of chemical research, Synopses,* 1978, vol. 7, 262-3
- *The Journal of Biological chemistry,* 1993, vol. 268, 6097-6106
- *Eur. J. Biochem.,* 1989, vol. 186, 389

SEQUENCE LISTING

<110> KANEKA Corporation

<120> PROCESS FOR PRODUCTION OF OPTICALLY ACTIVE 2-SUBSTITUTED PROPANAL DERIVATIVE

<130> B050353W001

<150> 2005-158393

<151> 2005-05-31

<160> 6

<170> PatentIn version 3.1

<210> 1

<211> 33

<212> DNA

<213> Artificial

<220>

<223> Primer1

<400> 1

cggtccagat atagaataaa tcatcatatt aag 33

<210> 2

<211> 29

<212> DNA

<213> Artificial

<220>

<223> Primer2

<400> 2

gaaatggtgc tacaaagtac ggttaacac 29

<210> 3

<211> 41

<212> DNA

<213> Artificial

<220>

<223> Primer3

<400> 3

atcgagctct aaggaggtta acaatgccat ttgttaagga c 41

<210> 4

<211> 32

<212> DNA

<213> Artificial

<220>

<223> Primer4

<400> 4

acgcgtcgac ttattaattt ttgtcccaac cg 32

<210> 5

<211> 44

<212> DNA

<213> Artificial

<220>

<223> Primer5

<400> 5

acgcgtcgac taaggaggtt aacaatgtat aaagatttag aagg 44

<210> 6

<211> 29

<212> DNA

<213> Artificial

<220>

<223> Primer6

<400> 6

gcgctgcagt tatccgcgtc ctgcttgga 29